(19) Europäisches Patentamt
European Patent Office
Office européen des brevets

(11) Veröffentlichungsnummer: **0 007 532**
**B2**

(12) # NEUE EUROPÄISCHE PATENTSCHRIFT

(45) Veröffentlichungstag der neuen Patentschrift: **07.11.90**

(51) Int. Cl.[5]: **B 01 F 17/22,** C 08 G 18/70, B 27 N 3/02, C 07 C 263/16

(21) Anmeldenummer: **79102432.6**

(22) Anmeldetag: **13.07.79**

(54) **Wässrige Isocyanat-Emulsionen, Verfahren zu deren Herstellung und deren Verwendung für die Verleimung von Holz.**

(30) Priorität: **19.07.78 DE 2831674**

(43) Veröffentlichungstag der Anmeldung: **06.02.80 Patentblatt 80/03**

(45) Bekanntmachung des Hinweises auf die Patenterteilung: **26.08.81 Patenblatt 81/34**

(45) Bekanntmachung des Hinweises auf die Entscheidung über den Einspruch: **07.11.90 Patentblatt 90/45**

(84) Benannte Vertragsstaaten: **AT BE CH DE IT NL SE**

(56) Entgegenhaltungen:
**DE-A-2 610 552**
**DE-A-2 703 271**
**DE-C- 853 438**

**H. Römpp: Chemie Lexikon, 6. Auflage, Stuttgart, (1973), pp. 3644-3645, 5080**

**Encyclopedia of Polymer Science and Technology, vol. 14, p. 244**

**Ullmanns Encyclopädie der technischen Chemie, 3rd Ed., vol. 14, (1963), p. 261-263, 293**

(73) Patentinhaber: **BASF Aktiengesellschaft**
**Carl-Bosch-Strasse 38**
**D-6700 Ludwigshafen (DE)**

(72) Erfinder: **Reuther, Wolfgang, Dr.-Chem.**
**Am Pferchelhang 16**
**D-6900 Heidelberg-Ziegelhausen (DE)**
Erfinder: **Segnitz, Adolph, Dr.-Chem.**
**Im Roehrich 46**
**D-6702 Bad Duerkheim 1 (DE)**
Erfinder: **Wittmann, Otto**
**Max-Beckmann-Strasse 7b**
**D-6710Frankenthal (DE)**
Erfinder: **Schatz, Hermann**
**Neubergstrasse 50**
**D-6730 Neustadt 15 (DE)**

(56) Entgegenhaltungen:

**Technische Information optal T 1528, Düsseldorf (Sichet-Werke) (1978)**
**Bayer AG: Anwendungstechnische Information Desmodur Leverkusen (1982)**
**Kirk Othmer: Encyclopedia of chemical technology, 2nd edition, vol. 8, p. 117-154 and vol. 19, pp. 507-593**

EP 0 007 532 B2

Courier Press, Leamington Spa, England.

**Beschreibung**

Die Verwendungen von Isocyanaten als Spanplattenbindemittel ist bekannt (vgl. z.B. H. J. Deppe und K. Ernst, Holz als Roh- und Werkstoff, Jahrg. 29, S. 45, 1971). Besonders bekannt sind Bindemittel auf der Grundlage technischer Gemische von Diphenylmethandiisocynaten. Als lösungsmittelfreie flüssige Produkte kann man sie in den in der Holzindustrie üblichen Verarbeitungsanlagen einsetzen.

Es ist jedoch ein Nachteil, insbesondere bei niedrigen Dosierungen, dass sich Bindemittel auf der Grundlage von Isocyanaten nur schwer auf dem Spanmaterial gleichmässig verteilen lassen. Dadurch können die technologischen Eigenschaften der fertigen Spanplatte hinsichtlich der Festigkeit verschlechtert werden. Auch ist nachteilig, dass sich mit Isocyanat verunreinigte Geräte bzw. Maschinenteile mit Wasser nicht ohne weiteres reinigen lassen. Es ist zwar bekannt, zur Verdünnung von Isocyanaten organische Lösungsmittel anzuwenden. Geeignete Lösungsmittel wie Dichlormethan u.a. sind aber aus toxikoligischen Gründen, andere Lösungmittel wie Acetale wegen der Feuergefährlichkeit praktisch nicht anwendbar.

In der bereits 1942 angemeldeten DE—B 853 438 ist beschrieben, zum Imprägnieren von Faserstoffen, Häuten und Fellen wäßrige Polyisocyanat-Dispersionen zu verwenden, die mit Emulgiermitteln wie Ethylenoxid-Addukten von höheren Fettalkoholen, Alkylphenolen, Sulfosäuren alkylierter Naphthaline, Ester höherer Fettsäuren, Leim, Polyvinylalkohol oder wasserlösliche Celluloseprodukte hergestellt werden können. Die angegebenen Emulgiermittel sind teils schlecht mit gleichbleibender Qualität herstellbar, teils reagieren sie mit den Polyisocyanaten. Die frühe Lehre hat nicht zu einer befriedigenden kommerziellen technischen Verwertung der Polyisocyanat-Dispersionen bei der Herstellung von Holzwerkstoffen wie Spanplatten geführt.

Es ist in der DE—OS 26 10 552 bzw. der FR—PS 23 03 661 der Vorschlag gemacht worden, Isocyanate, die als Bindemittel (Klebstoffe) für lignolcellulosehaltige Partikel in Frage kommen, als wässrige Emulsion zu verwenden. Allerdings müssen dabei — so das Ergebnis der dort abgehandelten Experimente — an die Isocyanate selbst und an die Emulgierhilfsstoffe besondere Anforderungen gestellt werden. Als (Poly)-isocyanate werden bevorzugt Präpolymere, d. h. Umsetzungsprodukte von eigentlichen Polyisocyanaten mit Polyester- bzw. Polyätherpolyolen, die noch Isocyanat-Endgruppen aufweisen, verwendet. Als Emulgatoren bzw. oberflächenaktive Mittel werden nichtionogene Produkte verwendet, die ausserdem frei sein sollen von Hydroxyl-, Amino- oder Carbonsäureestern. Offenbar ist es aber trotz beträchtlicher Mengen spezieller hydrophober Polyole, die zur Herstellung der o.g. Präpolymeren eingesetzt werden, nicht möglich, Emulsionen zu erhalten, die eine genügende Haltbarkeit aufweisen; es zeigt sich vielmehr, dass der Verlust an Isocyanatgruppen in der Emulsion im Verlaufe von jeweils zwei Stunden bei Raumtemperatur im günstigsten Falle etwa 14 bis 17%, in ungünstigeren Fällen über 50% beträgt.

Für den Fachmann auf dem Gebiet der Herstellung von Holzwerkstoffen ergibt sich daraus, dass ein solches Bindemittel ohne praktischen Wert ist, weil die technisch notwendigen Wartezeiten zwischen dem Ansetzen einer Emulsion und dem Verpressen, die durch die Kapazität der Vorratsbehälter für Leim und beleimtes Spänegut gegeben sind, nicht erreicht werden.

Es besteht daher die Aufgabe, wässrige Isocyanat-Zubereitungen zu schaffen, die die erwähnten Nachteile nicht oder in wesentlich geringerem Masse besitzen, d. h. die Schaffung lagerfähiger wässriger Emulsionen von Polyisocyanaten.

Die Erfindung betrifft die Hestellung wässriger ausreichend lagerfähiger Polyisocyanat-Emusionen dadurch, dass man die Polyisocyanate in Gegenwart eines Emulgators auf der Grundlage eines durch Polymerisation eines olefinisch un gesättigten Carbonsäureamids oder durch polymeranaloge Umsetzung anderer Vinylpolymerisate gebildeten wasserlöslichen polymeren carbonsäureamids in Wasser dispergiert bzw. emulgiert.

Das Ergebnis der Erfindung ist überraschend da — nicht zuletzt aus den genannten Druckschriften — bekannt ist, dass gerade aromatische Isocyanate mit Wasser schnell reagieren; lagerfähige Emulsionen solcher Isocyanate sind bisher nicht beschrieben worden.

Beispiele von organischen Polyisocyanaten, welche bei dem erfindungsgemässen Verfahren verwendet werden können, sind aromatische Isocyanate, insbesondere Diisocyanate wie m- und p-Phenylendiisocyanat, Tolylen-2,4- und -2,6-diisocyante, Diphenylmethan-4,4'-diisocyanat, Chlorphenylen-2,4-diisocyanat, Naphthylen-1,5-diisocyanat, Diphenylen-4,4'-diisocyanat, 4,4'-Diisocyanat-3,3'-dimethyldiphenyl, 3-Methyldiphenylmethan-4,4'-diisocyanat, Diphenyläther-diisocyanat und Triisocyanate, wie 2,4,6-Triisocyanatotoluol und 2,4,4'-Triisocyanatdiphenyläther. Sie werden i. a. durch Phosgenierung entsprechender Amine in bekannter Weise erhalten. Mischungen von Isocyanaten sind beispielsweise die im Handel verfügbaren Mischungen von 2,4- und 2,6-Isomeren des Tolylendiisocyanats sowie die Mischungen von Di- und höheren Polyisocyanaten, welche durch Phosgenierung von Anilin-Formaldehyd-Kodnensaten hergestellt werden. Solche Mischungen sind in der Praxis allgemein bekannt und sie schliessen die rohen Phosgenierungsprodukte ein, welche Mischungen von Methylenbrücken aufweisenden Polyphenylpolyisocyanaten, einschliesslich Diisocyanat, Triisocyanat und höhere Polyisocyanate zusammen mit den Phosgenierungsnebenprodukten enthalten.

Bevorzugte Polyisocyanate, welche erfindungsgemäss verwendet werden können, sind diejenigen, worin das Isocyanat ein aromatisches Diisocyanat oder Polyisocyanat mit höherer Funktionalität ist, insbesondere technische Mischungen von Methylenbrücken aufweisenden Polyphenylpolyisocyanaten, welche Diisocyanate, Triisocyanate oder Polyisocyanate mit höherer Funktionalität enthalten. Methylenbrücken aufweisende Polyphenylpolyisocanate sind allgemein bekannt, und sie besitzen die allgemeine Formel

I

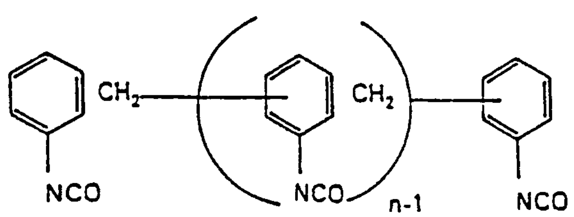

worin n mehr als 1 ist und im Falle der technischen Mischungen ein Mittel von mehr als 1 darstellt. Diese Verbindungen werden hergestellt durch Phosgenieren von entsprechenden Mischungen von Polyaminen, wie sie durch Kondensation von Anilin und Formaldehyd erhalten werden. Es ist üblich, technische Mischungen von Methylenbrücken aufweisenden Polyphenylpolyisocyanaten, welche Diisocyanat, Triisocyanat und Polyisocycnate mit höherer Funktionalität enthalten, als MDI zu bezeichnen.

Weniger geeignete organische Isocyanate, welche erfindungsgemäss verwendet werden können, sind Isocyanatreste aufweisende Vorpolymere, die hergestellt sind durch Umsetzen eines Überschusses von Diisocyanat oder einem Polyisocyanat mit höherer Funktionalität mit einem Hydroxylenden aufweisenden Polyester oder Hydroxylenden aufweisenden Polyäther sowie Produkte, welche durch Umsetzen eines Überschusses von Diisocyanat oder Polyisocyanat höherer Funktionalität mit monomerem Polyol oder einer Mischung von monomeren Polyolen, wie Äthylenglykol, Trimethylolpropan und Butandiol erhalten werden.

Es ist allerdings möglich, dass sich solche Vorpolymere (Präpolymere) im Verlauf des erfindungsgemässen Herstellverfahrens in geringen Mengen bilden.

Im allgemeinen ist eine Menge von etwa 0,5 bis 5% des wasserlöslichen Polycarbonsäureamids (z.B. Polyvinylpyrrolidon), bezogen auf das gesamte Emulsionsgewicht, ausreichend, um eine Emulsion herzustellen, deren titrierbarer Gehalt an Isocyanatgruppen in 10 Stunden um nicht mehr als etwa 1% abfällt. Die nachstehende Tabelle, die mit der Emulsion des Herstellbeispiels aufgestellt wurde, zeigt dies.

Polyvinylpyrrolidon eines geeigneten Molgewichtsbereiches (z.B. von 20 000 bis 1 000 000) ist handelsüblich und bedarf keiner näheren Erläuterung. Es wird bevorzugt dem Emulgierwasser in entsprechender Konzentration zugesetzt und lässt eine hohe Emulgiergeschwindigkeit zu.

Andere polymere Carbonsäureamide, die durch übliche Polymerisation von olefinisch ungesättigten Monomeren erhalten werden, sind z.B. die Polymeren des Vinylcaprolactams, des Acrylamids, Methacrylamids. Sie können z. T. auch durch polymeranloge Umsetzung anderer Vinylpolymerisate, z.B. durch Verseifen aus Polyacrylnitril erhalten werden.

In manchen Fällen ist die Verwendng von mehreren Emulgatoren nebeneinander von Vorteil. Ebenfalls empfehlenswert ist die zusätzliche Verwendung z.B. von Schutzkolloiden. Da in einzelnen Fällen durchaus ungewiss ist (bei Verwendung mehrerer emulgierender Mittel), welchs der Mittel als Emulgator und welches als Schutzkolloid anzusehen ist, soll die Erfindung in ihrem weitesten Sinne so verstanden werden, dass die Verwendung auch geringerer, wirksamer Mengen des Polyamids zusammen mit einem anderen emulgierenden Mittel, das auch in höherer Menge vorliegen kann, hiervon umfasst sein soll.

Als andere emulgierende Mittel kommen z.B. höhermolekulare Polyglykole, insbesondere Polyäthylenglykole, Polypropylenglykole sowie gemischte Glykole mit Molekulargewichten zwischen 4 000 und 20 000, vorzugsweise zwischen 6 000 und 12 000 in Betracht.

Die Emulsionen enthalten z.B. 5 bis 70% Polyisocyanat, eine ausreichende Menge Emulgator und im übigen Wasser. Sie werden in der für Emulsionen üblichen Weise durch Verrühren der Bestandteile mit geeigneten Vorrichtungen erhalten.

Zur Unterscheidung der Erfindung von dem, was schon bekannt ist, muss im Zusammenhang mit der Herstellung der Emulsion auf folgendes hingewiesen werden:

Da es bekannt ist, dass hydroxylgruppenhaltige Verbindungen mit Isocyanaten reagieren, sollen solche Verbindungen nur in solchen Mengen mitverwendet werden, dass die vorhandenen Hydroxylgruppen nur einen Bruchteil, z.B. nicht mehr als 10 bis höchstens 40% der zur Verfügung stehenden Isocyanatgruppen ausmachen.

Die Stabilität von Isocyanat-Emulsionen kann nach bekannten Verfahren, z.B. durch Titration gegen Bromphenolblau gemessen werden. Hierzu werden in bestimten Zeitabständen Proben der Emulsion entnommen, mit überschüssiger Dibutylamin-Lösung versetzt und nicht verbrauchtes Amin mit methanolischer Salzsäure zurücktriert. Als Kennzahl "R" wird das Verhältnis gewählt:

$$R = \frac{\text{mval Dibutylamin}}{\text{g Diisocyanat}}$$

Zur Auswertung der Stabilität wird der R-Wert von reinem Isocyanat bestimmt und gleich 100% gesetzt. Die R-Werte der wässrigen Isocyanat-Emulsion werden laufend gemessen und die Abnahme jeweils gemittelter R-Werte (in % vom Anfangswert) als Ordinate gegen die Zeit als Abszisse aufgetragen.

Die den Beispielen beigegebenen Tabellen zeigen die Abnahme des Isocyanat-Gehaltes bei

einer Temperatur von 23°C in Anhängigkeit von der Zeit.

Beispiel 1

Eine typische Emulsion wird aus 50 Teilen technischem Diphenylmethandiisocyanat (MDI) und 50 Teilen 4%iger wässriger Emulgator-Lösung in folgender Weise erhalten:

Es wird eine 4%ige wässrige Lösung von Polyvinylpyrrolidon hergestellt, indem das unter dem Handelsnamen Luviskol K 90 erhältliche) Polyvinylpyrrolidon warm in Wasser gelöst wird. In die abgekühlte Lösung wird Diphenylmethan-4,4'-diisocyanat eingerührt. Das Emulgieren mittels eines hochtourigen Rührwerks erfordert ca. 10 bis 30 Sekunden.

Die Emulsionen sind praktisch innerhalb von 7 Stunden stabil. Die Schwankungen liegen im Bereich der Fehlergrenze der Messmethode.

TABELLE 1

| Stunden | Isocyanat (%) bezogen auf Nullwert |
|---|---|
| 0 | 100,0 |
| 1 | 100,6 |
| 2 | 100,0 |
| 3 | 99.4 |
| 4 | 99,4 |
| 5 | 98,7 |
| 6 | 100,0 |
| 7 | 99,4 |

Beispiel 2

Eine Emulsion wird aus 50 Teilen technischem Diphenylmethandiisocyanat (MDI) und 50 Teilen 1%iger wässriger Emulgator-Lösung wie folgt erhalten:

Es wird eine 1%ige wässrige Lösung eines Emulgator-Schutzkolloid-Systems hergerstellt, indem man Polyäthylenglykol das unter dem Handelsnamen Pluriol E 9000 erhältliche Produkt) und Polyvinylpyrrolidon (das unter dem Handelsnamen Luviskol K 90 erhältliche Produkt) im Gewichtsverhältnis 19:1 warm in Wasser löst. In die abgekühlte Lösung wird Diphenylmethandiisocyanat eingerührt. Das Emulgieren mittels eines hochtourigen Rührwerks erfordert ca. 10 bis 30 Sekunden.

Die Emulsionen weisen innerhalb von 9 Stunden innerhalb des Messfehlers keinen Verlust an Isocyanat-Aktivität auf.

TABELLE 2

| Stunden | Isocyanat (%) bezogen auf Nullwert |
|---|---|
| 0 | 100,0 |
| 1 | 100,6 |
| 2 | 100,0 |
| 3 | 100,0 |
| 4 | 100,0 |
| 5 | 99,4 |
| 6 | 98,1 |
| 7 | 100,0 |
| 8 | 99,4 |
| 9 | 99,4 |

Beispiel 3

Wie vorstehend beschrieben, wird unter Verwendung einer 3%igen wässrigen Emulgator-Lösung eine Emulsion hergestellt. Die 3%ige wässrige Emulgator-Lösung wurde erhalten, indem man Polyäthylenglykol und unter dem Handelsnamen Lubasin S erhältliches Polyvinyl-ε-caprolactam im Gewichtsverhältnis 30:1 bei Raumtemperatur in entsprechender Menge Wasser löst.

Emulsionen aus 50 Teilen Diphenylmethan-4,4'-diisocyanat und 50 Teilen 3%iger wässriger Emulgator-Lösung sind 6 Stunden ohne messabaren Abfall der Isocyanat-Aktivität haltbar.

Anwendungsbeispiel

Mit der Emulsion des Beispiels 1 und einer etwas weniger konzentrierten Zubereitung werden Spanplatten hergestellt und ihre technologische Anwendung geprüft. Hierzu werden Fichtenspäne, die unter Technikumsbedingungen hergestellt worden waren und einen Feuchtigkeitsgehalt von ca. 4% haben, in einer üblichen Beleimungsvorrichtung (Drais-Mischer) mit den Klebstoffmischungen behandelt. Als Hydrophobiermittel dient eine 50%ige Parrafinemulsion; die Menge beträgt in allen Fällen 1% Festwachs auf absolut trockene Späne. Zur Einstellung eines Feuchtigkeitsgehaltes der beharzten Späne von ca. 12% wird jeweils die notwendige Menge an Wasser vor der Beharzung auf die Späne gesprüht.

Die Klebstoffemulsion wird mittels eines Mischers so auf den Spänen verteilt, dass jeweils 4% Isocyanat, bezogen auf den Holzanteil, verwendet werden. Als Vergleich wird der Versuch mit nichtemulgiertem Isocyanat durchgeführt.

Die so vorbehandelten Späne werden in einer Versuchsschüttanlage zu einem Spankuchen

geformt. Die Presstemperatur beträgt 165°C bei einer Presszeit von 4 Minuten und einem spezifischen Druck von 2,5 $N/mm^2$. Wegen der Neigung der Isocyanate, an den Presskuchen anzuhängen, wird mit Trennpapieren gearbeitet. Die Dicke der gefertigten Spanplatten beträgt 18,5 mm, die Dichte ca. 610 $kg/m^3$.

| | Isocyanat | 40%ige Emulsion | 50%ige Emulsion |
|---|---|---|---|
| Plattenprüfung nach DIN 52 360—65 | | | |
| Biegefestigkeit ($N/mm^2$) | 30,7 | 31,1 | 32,1 |
| Querzugfestigkeit V 20 ($N/mm^2$) | 0,78 | 1,02 | 1,02 |
| Querzugfestigkeit V 100 ($N/mm^2$) | 0,23 | 0,30 | 0,28 |
| Quellung nach 2 h (%) | 4,1 | 4,3 | 4,2 |
| Quellung nach 24 h (%) | 17,1 | 16,3 | 16,3 |

**Patentansprüche**

1. Verfahren zur Herstellung wäßriger Polyisocyanat-Emulsionen, dadurch gekennzeichnet, daß man das Polyisocyanat in Gegenwart eines Emulgators auf der Grundlage eines durch Polymerisation eines olefinisch ungesättigten Carbonsäureamids oder durch polymeranaloge Umsetzung anderer Vinylpolymerisate, z.B. durch Verseifen von Polyacrylnitril, gebildeten wasserlöslichen polymeren Carbonsäureamids in Wasser dispergiert bzw. emulgiert.

2. Verfahren nach Anspruch 1, dadurch gekennzeichnet, daß der Emulgator Polyvinylpyrrolidon ist.

3. Verfahren nach Anspruch 1, dadurch gekennzeichnet, daß man als Polyisocyanat das technische Gemisch von diphenylmethan-diisocyanaten verwendet, wie es durch Phosgenierung der Umsetzungsprodukte von Anilin und Formaldehyd erhalten wird.

4. Wäßrige Polyisocyanat-Emulsionen, dadurch erhältlich, daß man das Polyisocyanat in Gegenwart eines Emulgators auf der Grundlage eines durch Polymerisation eines olefinisch ungesättigten Carbonsäureamids oder durch polymeranaloge Umsetzung anderer Vinylpolymerisate, z.B. durch Verseifen von Polyacrylnitril, gebildeten wasserlöslichen polymeren Carbonsäureamiden in Wasser dispergiert bzw. emulgiert.

5. Wäßrige Polyisocyanat-Emulsionen nach Anspruch 4, dadurch gekennzeichnet, daß der Emulgator Polyvinylpyrrolidon ist.

6. Verwendung von Polyisocyanat-Emulsionen nach Anspruch 4 oder 5 zur Verleimung von Holz bzw. zur Herstellung von Holzwerkstoffen, insbesondere Spanplatten.

**Revendications**

1. Procédé de préparation d'émulsion d'émulsions de polyisocyanates aqueuses, caractérisé en ce que l'on émulsionne ou disperse le polyisocyanate dans l'eau en présence d'un émulsif à base d'un amide d'acide carboxylique polymère, soluble dans l'eau, formé par la polymérisation d'un amide d'acide carboxylique insaturé, ou par la réaction analogue à celle de polymères d'autres polymères vinyliques, par exemple, par saponification du polyacrylonitrile.

2. Procédé suivant la revendication 1, caractérisé en ce que l'èmulsif est la polyvinylpyrrolidone.

3. Procédé suivant la revendication 1, caractérisé en ce que l'on utilise, à titre de polyisocyanate, le mélange industriel de diisocyanates de diphénylméthane, tel qu'on obtient par la phosgénation des produits de la réaction de l'aniline et du formaldéhyde.

4. Emulsions de polyisocyanates aqueuses que l'on peut obtenir par le fait que l'on émulsionne ou disperse le polyisocyanate dans l'eau en présence d'un émulsif à base d'un amide d'acide carboxylique polymère, soluble dans l'eau, formé par la polymérisation d'un amide amide d'acide carboxylique insaturé, ou par la réaction analogue à celle de polymères d'autres polymères vinyliques, par exemple, par saponification du polyacrylonitrile.

5. Emulsions de polyisocyanates aqueuses suivant la revendication 4, caractérisé en ce que l'émulsif est la polyvinylpyrrolidone.

6. Utilisation d'émulsions de polyisocyanates suivant les revendications 4 et 5 pour le collage du bois, ou la fabrication de matériaux à base de ou dérivés du bois, en particulier, des panneaux de copeaux.

**Claims**

1. A process for the preparation of an aqueous polyisocyanate emulsion, wherein the polyisocyanate is emulsified or dispersed in water in the presence of an emulsifier based on a water-soluble polymeric carboxamide formed by polymerization of an olefinically unsaturated carboxamide or by polymer-analogous reaction of other vinyl polymers, for example by hydrolysis of polyacrylonitrile.

2. A process as claimed in claim 1, wherein the emulsifier is polyvinylpyrrolidone.

3. A process as claimed in claim 1, wherein the polyisocyanate used is the industrial mixture of diphenylmethane diisocyanates, as obtained by phosgenation of the reaction products of aniline and formaldehyde.

4. An aqueous polyisocyanate emulsion, obtainable by emulsifying or dispersing the polyisocyanate in water in the presence of an emulsifier based on a water-soluble polymeric carboxamide formed by polymerization of an olefinically unsaturated carboxamide or by polymer-analogous reaction of other vinyl polymers, for example by hydrolysis of polyacrylonitrile.

5. An aqueous polyisocyanate emulsion as claimed in claim 4, wherein the emulsifier is polyvinylpyrrolidone.

6. Use of a polyisocyanate emulsion as claimed in claim 4 or 5 for gluing wood or for the production of woodworking materials, in particular particle boards.